Europäisches Patentamt

European Patent Office

Office européen des brevets

(11) Publication number: **0 360 929**
**A1**

## (12) EUROPEAN PATENT APPLICATION

(21) Application number: 88308175.4

(22) Date of filing: 02.09.88

(51) Int. Cl.⁵ **A61F 13/00 , A61F 13/15**

(43) Date of publication of application:
**04.04.90 Bulletin 90/14**

(84) Designated Contracting States:
**BE DE FR GB**

(71) Applicant: **VERATEC, INC.**
**95 West Street**
**Walpole, MA 02081(US)**

(72) Inventor: **Karami, Hamzeh**
**1630 Worcester Road**
**Framingham Massachusetts, 01701(US)**
Inventor: **Parker, Duane A.**
**24 Colonial Drive**
**Somers Connecticut, 06701(US)**

(74) Representative: **Johnson, Terence Leslie et al**
**Edward Evans & Co. Chancery House 53-64**
**Chancery Lane**
**London WC2A 1SD(GB)**

(54) **Fibre-film substrate.**

(57) A fiber-film substrate (10) to be used as a coversheet on disposable articles, comprising at least one layer of a polyethylene thermoplastic film (14) extending over and secured to a surface of at least one layer of a uniformily thick nonwoven polypropylene fibrous web (12), by means of a heated embossed roll applied to a surface of the thermoplastic film. The heat from the roll causes the film to melt in discrete areas forming apertures (16) therein without affecting the fibers or the uniform thickness of the fibrous web itself. As the film melts, it contacts fibers in the unaffected adjacent fibrous web layer, causing the film and web to be secured to each other at least at the peripheral edges of the discrete apertured areas.

FIG. 3

EP 0 360 929 A1

**FIBER-FILM SUBSTRATE**

Background of the Invention

This invention relates generally to coversheets for such absorptive devices as disposable diapers. sanitary napkins. disposable bed pads, nursing pads, finger dressings. and incontinent diapers. or the like.

Usually. absorptive devices consist of a coversheet. an absorbent pad and a barrier sheet in close contact with each other.

Prior art coversheets usually consist of a flexible apertured plastic sheet to prevent liquid absorbed in an absorbent pad. within an absorptive device. from striking through and soiling outer adjacent clothing. The apertured waterproof plastic sheet of the prior art does provide some degree of dryness. In order for an absorptive device to be acceptable to the public. specifically women. liquid excreted by the body must be capable of being transferred through the coversheet. which is in intimate contact with the body. away from the body into an absorbent pad as quickly as possible. Thus, this quick transmital of liquid through a coversheet is an essential requirement in developing an absoptive device. In addition, the prior art plastic sheets do not adhere or come into intimate contact with an absorbent pad. Thus. this may result in the absorbent pad shifting within the device thereby causing

In U.S. Patent Number 3.881,489. there is provided a disposable absorptive device having an absorptive body enclosed within a top. coversheet and an improved breathable liquid impervious backsheet. The topsheet may be any conventional top sheet. such as described in U.S. Patent Reissue No. 26,151. The improved backsheet comprises the combination of two distinct adjacent hydrophobic layers to form an effective breathing portion of the backsheet. The first layer being liquid permeable and having a low void volume while the second layer is liquid permeable and having a higher void volume than the first.The combination of the two backsheet layers prevent the passage of liquid while permitting the passage of gases therethrough. A disadvantage associated with the aforementioned prior art is that the topsheet is a typical topsheet made from a plastic film without any distinguishing features, such as dryness or fluid penetration.

In U.S. Patent Number 3,292,619. there is described an invention directed to a non-adherent dressing in which only portions of the surface of the body side thereof are in contact with the body when applied to cover a wound therein. Only a minor portion of the total surface area of the body side of the dressing is in contact with the body. The major sites of fluid intake into the absorbent pad. in accordance with that invention. are located in depressions in the surface of the dressing which is in contact with the body.The walls of the depression are sloped inwardly into the body of the dressing and are lined with a thin film which covers the surface of the pad at the body side of the dressing. The film lining the walls of the depressions is perforated. Each depressed film portion containing a plurality of openings into which the fluid passes. A disadvantage of this prior art fabric is that it is bonded together by passing it between the nip of an embossed roll and a smooth surface roll which imparts depressions to the fabric. The depressions in the fabric thus reduce the total area that is available in the absorbent material for absorbing fluid.

In U.S. Patent 3,331,728, there is described a film-fiber laminate that is made as a coversheet to cover absorbent material, such as that used in a sanitary napkin. The laminate is made in a continuous process with the film, as formed by a film extruder, being laid immediately onto an underlying perforated fiber web. Suction is then applied to the underside of the perforated web, with the freshly extruded film still in the highly tacky fluid state, to secure intimate bonding between the film and the underlying fibrous web, while at the same time rupturing the film in the areas immediately overlaying the openings in the fiber web, causing openings to be completely through the fabric. A disadvantage of the aforementioned prior art is that the openings in the composite are through the film and the fibrous web. This allows for ingress of fluid to pass through the composite into an absorbent material, but also allows for egress of the fluid, thus presenting a problem in containing fluid in a product, such as a sanitary napkin.

The aforementioned disadvantages in the prior art are not present in this invention because it is an integral composite made in one process step, having apertures in the film cover and no depressions nor holes disposed in the fibrous web. In addition to it being more economical to produce, it has qualities that are not present in the prior art which will be more evident in the remainder of this specification, drawings and claims.

## Summary of the Invention

A fiber-film laminate to be used as a coversheet on disposable articles. comprising at least one layer of an apertured polyethylene thermoplastic film extending over and secured to a surface of at least one layer of a uniformly thick nonwoven polypropylene fibrous web. Securing of the film to the fibrous web is accomplished when a heated embossed roll having lands and grooves is applied to a surface of the thermoplastic film. The heated lands of the roll, as they barely contact the surface of the film. causes the film to melt in discrete areas forming apertures in the film, and not the fibrous web. As the film melts and forms apertures. the melted film contacts the adjacent fibrous web layer in discrete areas, causing the film and web to be secured to each other at least at the peripheral edges of the discretely apertured areas.

An object of this invention is to provide a substrate having excellent tensile strengths. fluid penetration and rewet properties.

Another object of this invention is to provide a coversheet having minimal fluid retention capacity.

Still another object of this invention is to provide a unitary two sided sheet. one side an apertured film having a smooth and comfortable surface and on the opposite side a fibrous surface that has sufficient roughness to stay in intimate contact with an absorbent material adjacent thereto.

## Brief Description of the Drawing

Figure 1 is a view of the fiber-film composite illustrating the two layers of said composite.

Figure 2 is a prospective view of the composites to illustrate the apertures within the film.

Figure 3 is a cross-sectional view taken across the apertures of Figure 2 to illustrate that the apertures do not penetrate through the fibrous web material.

Figure 4 shows the film and the fibrous web as they are passed between a heated embossed roll and a smooth roll.

## Description of the Preferred Embodiment

The goal of prior art has been to develop a substrate having excellent tensiles; minimal fluid holding capacity; fluid penetration and rewet properties that is to be used as a coversheet on disposable articles, such as diapers and sanitary napkins.

To acheive most of these goals the prior art has used separate layers to make up a disposable article. These layers usually consist of a layer of plastic film as a coversheet and a layer of absorbent material encased in said coversheet. When substrates are used as a coversheet on a sanitary napkin, the top surface must not hold any of the menstrual fluid and must have minimal stain in order to be well accepted by women. Another requirement is to have a substrate that has one side with a smooth and comfortable surface and the another side having a surface with enough roughness to stay in intimate contact with an absorbent pad in a sanitary napkin to hold the absorbent pad in place thus facilitating the transmission of fluid from the apertured substrate into the absorbent pad.

The present invention has acheived what the prior art has been unable to do by providing a unitary two-sided substrate with the aforementioned properties. In addition to hygenic products, this invention may be used, as a substrate for a tape backing, tea bags, filtration membranes, wipes, sponges, finger dressings, milk filters or filters for wine, beer, oil etc..

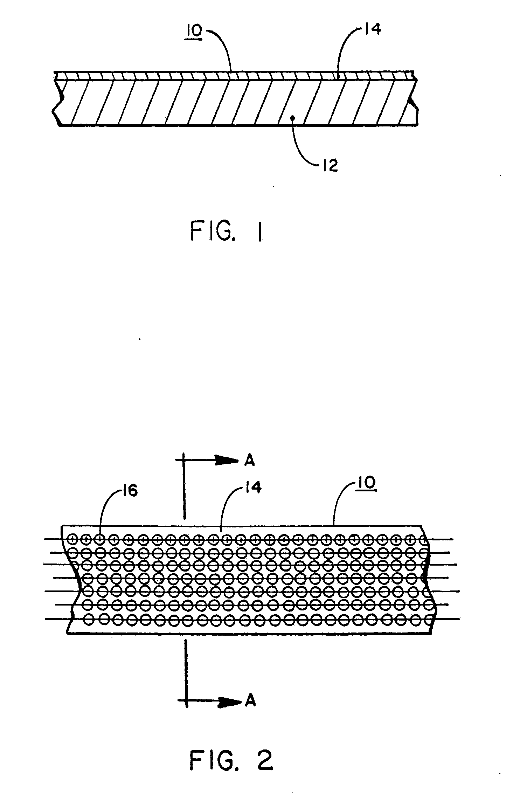

The present invention substrate 10, as shown in Figure 1, may be made of at least one layer of polypropylene fibers 12, having a uniform thickness, secured to at least one layer of, for example, thermoplastic polyethylene film 14. Although the aforementioned construction is the preferred embodiment, the film layer may also be made from any thermoplastic film, such as polypropylene, polyurethane, polyester, co-polyester or co-polypropylene. The fibrous layer may be made from any synthetic fiber, such as polyolefin, Rayon, polyester, or acrylic. or natural fibers, such as cotton, wood pulp, or any combination of synthetic and natural fibers. In addition, the nonwoven fibrous web could be made of staple fibers, continuous filaments or microfibers.

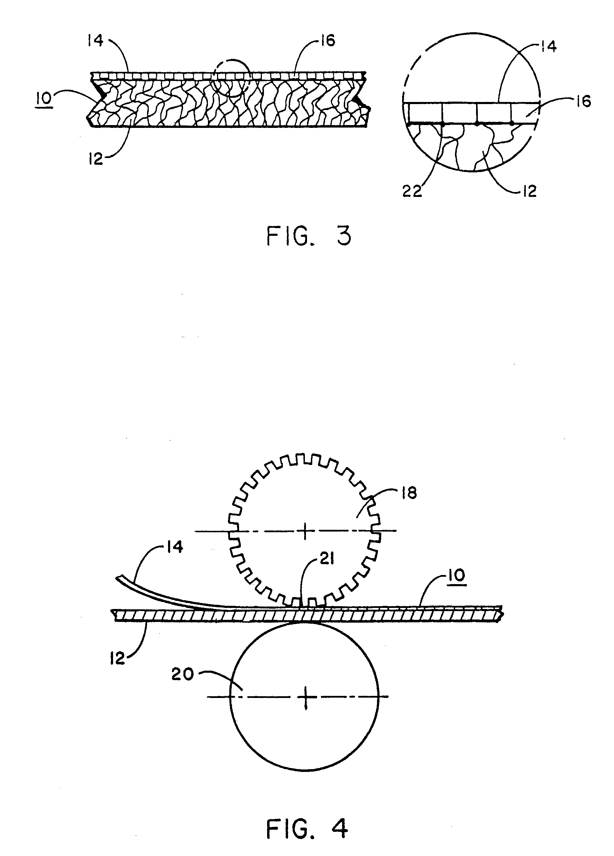

As shown in Figure 4, the film layer 14 is positioned above the fibrous web layer 12. The film layer 14 and fibrous web layer 12 are then fed between two mechanically driven heated rolls. The top roll being an embossed roll 18, having lands and grooves, and the bottom roll being a smooth roll 20. The smooth roll 20 may be cooled to maintain a constant temperature. The embossed roll 18 may have varying patterns. depending on the aperture requirements. The embossed roll 18 is set at a position whereby the raised lands of the roll barely makes contact with a surface of said film 14 As the heated lands 21 of the roll 18 contacts a surface of the film. the heat melts the film in discrete areas. As the film melts in these discrete areas. apertures 16 are created or formed in the film 14. as shown in Figure 2. In addition. as the melted film 22 flows. it contacts fibers in the fibrous layer, as shown in the insert of Figure 3. Then. as the fiber-film layered substrate exits the nip of the heated rolls. the melted film cools and the layers become secured to each other in the discrete apertured areas, also shown in the insert. at least at the peripheral edges of those apertured areas. It is important to note that the heated lands 21 of roll 18 do not affect the structure or composition of the fibrous layer in any manner. The heated lands 21 come in contact with only the surface of the film 14: it does not pentrate beyond that point. Thus the fibers of the fibrous web are substantially unaffected and the fibrous web retains its uniform thickness. In addition. because the heated roll barely contacts the film it does not come into contact with the fibrous web. thus there are no depressions or holes in the fibrous web, as may appear in prior art fabrics. As a result of securing in this manner. the present invention substrate has two sides, one side a surface which has a film with apertures and the other side a fibrous layer, as illustrated in Figure 3, which does not contain apertures. The shape and size of the apertures in the film may vary depending upon the application of the substrate, but the film should have an open area of at least 1% of the total surface area of the film. The open area being defined as the apertures. The preferred apertured area is 45% of said surface area. The apertures may cover a majority of the surface of the film or they may be positioned in zones depending on the requirements of the product the present invention is used on. The film thickness should be at least 0.1 mil in order to cover the fibrous surface and simultaneously yield a smooth surface. Even though at least 0.1 mil is preferred, the thickness of the film may range between 0.1 to 15 mils. Although it is preferred to use film that has been extruded and cured. freshly extruded film, still in a heat softened tacky state. may be placed directly on the fibrous web. thus inhancing and improving the securing of the film to the web. The fibrous layer may be made from many different fiber types and quantities depending on the end use. It is preferred to utilize a blend of synthetic and natural hydrophillic and hydrophobic fibers in order to minimize the fluid holding capacity of the substrate. The weight of the fibrous web may vary between 4 to 100 grams per square yard (gyd2) depending on the product design. The thickness of the two layers together may vary depending on the required physical properties, such as machine and cross direction tensile strengths. fluid holding capacity, liquid penetration, etc. The two layers, as shown in Figure 3, may be secured together with the fibers having varying degrees of fiber to film bonding, depending on the pressure and temperature used in the process.

Surface treatment embossing is important and may be any continuous or discrete patterns with varied dimensions. The preferred method of aperturing is by the technique described in U. S. Patent No. 3,507,943. of common assignee, incorporated by reference herein and which discloses the use of grooved rolls for bonding and aperturing. However, other methods, such as the use of engraved male and female rolls. engraved and smooth rolls, or endless belts may also be used. In the case of endless belts, hot air and a vacuum may be used to form the apertures and bond the layers. Another method of aperturing thermoplastic film is described in U. S. Patent No. 3,038,198. of common assignee, incorporated by reference herein.

The present invention as described herein is of particular advantage when used in disposable articles, such as sanitary napkins, because it will provide excellent softness, dryness, fluid penetration and other physical properties not available in prior art coversheets. When using the present invention coversheet on disposable articles, especially a sanitary napkin, which has an inner absorbent core or pad, the strike-through values or fluid penetration rate may be controlled without the need to attach the present invention coversheet onto the absorbent pad as is done in prior art. The term "strike-through" is defined as the time it takes a given amount of liquid to pass through a coversheet into an absorbent material. The less time it takes for the liquid to pass through the coversheet the better the strike-through value. To improve strike-through values, prior art disposable articles, such as sanitary napkins, have an adhesive layer, which totally covers the coversheet, and is between the coversheet and the absorbent pad layer. This additional layer was to insure good contact between the coversheet and the absorbent material. This contact is necessary because the fibers of the absorbent pad have to be as close as possible to the coversheet in order to wick fluid, that is on the coversheet, through it. No intermediate layer is necessary in the present invention. substrate. because the fibrous layer described herein is an integral part of the coversheet, therefore no closer contact can exit between a coversheet and an absorbent material. Furthermore, the fibrous layer does an excellent job of wicking any fluid on the hydrophobic film layer into it. The fluid is then transfered, by wicking, from the fibrous layer into a hydrophilic absorbent pad. This is a significant advance and a definite advantage over the prior art. Also, by varying the amount of hydrophilic fibers in the fibrous web, the strike-through value of the present invention may be controlled, and the substrate may have varying degrees of fluid penetration, by the choice of the fibers used in the fibrous layer. For example, a mixture of hydrophilic and hydrophobic fibers, such as acrylic and polypropylene fibers, or cotton and polypropylene fibers . In addition,because of the wicking action of the hydrophilic fibers into an adjacent absorbent pad, fluid is drawn from the fibrous layer into the absorbent material. Thus, there is minimal fluid retained in the coverstock substrate of this invention. Another advantage of the present invention, which is due to the fact that the film of the present invention is apertured without penetrating the adjacent web. Thus, the web layer having no apertures, and when secured to the film, acts as a buffer between the film layer and absorbent pad, thus preventing particles of absorbent material from penetrating the film and coming in contact with the skin of the wearer. Because the web is constructed of fibers its surface has a tendency to be fuzzy. This surface fuzziness gives an additional advantage to the present invention, in that the fuzzy surface attaches very easily to other fibrous surfaces, such as an absorbent pad, thus securing the pad and coversheet together, eliminating any shifting of the absorbent pad within the sanitary napkin, when being worn. The coversheet therefore is more stable on an absorbent pad, resulting in comfort and good pad stability. Still another advantage that the present invention has is the reduction of rewet or the dryness of the surface of coversheets in products using this invention.Rewet and dryness, for the purpose of this specification, are synonomous. Rewet may be defined as the amount of fluid, which will tend to flow from an absorbent pad back towards the outer face of the coversheet, when the pad is completely saturated.Thus, the less fluid to reach the surface of the coversheet, the dryer that surface remains. The present invention reduces rewet in two ways. First, by the hyrophilic fibrous layer slowing down the movement of the fluid by re-absorbing the fluid from the absorbent pad as the fluid moves outward from the pad. Secondly, when the fluid has moved through the fibrous layer, it comes in contact with the hydrophobic film layer and because of the resulting surface tension of the fluid with the film, a substantial amount of fluid will not pass outward through the apertures in the film.

The present invention substrate will have a smooth and woven appearing surface, due to the affect of the embossing roll on the film side. In addition, the fibrous side when secured or bonded to the film will provide support to the film. The substrate will also provide softness and comfort because of the smoothness of the surface on the film side and the cushioning effect of the fibrous side. The present invention substrate will have a textile appearance due to the apertures and the many different shapes, depth, etc. of micro embossing on the film surface, that may be used. For example, the embossed pattern may be pointed, circular, lines, etc. Also, by varying the percent open area of the apertures in the film, the ratio of strike-through to dryness may be changed. However, the more apertures in the film, which does give a better strike through, the greater the chance of rewet and wetting occuring in the coversheet, which is a definite disadvantage. Thus the preferred apertured area is 45% of the surface area. The optimum condition, as used in the present invention, is to have a sufficent amount of apertures in the coversheet as to give good fluid penetration or strike-through, while retaining the dryness on the coversheet surface.An important property of this present invention substrate is that the substrate may have varying degrees of dryness by using an appropriate fibrous layer and film thickness.

The present invention also has excellent tensile strength, which is due to the fact that it has a plastic film incorporated within it.

To illustrate that the present embodiment has either superior or substantially equal properties to that of the prior art, a comparison test, to compare strike-through and rewet values of different coversheets was conducted. Two identical hygenic sanitary napkins, manufactured by Proctor and Gamble under the Trade name "Always" were used herein for comparison purposes. The coversheet of the Proctor and Gamble napkin is attached to the absorbent material, in the napkin, by hydrogen bonding. To make the comparison test one napkin, hereinafter referred to as "A",was tested with the coversheet as manufactured by Proctor and Gamble, while the other napkin, hereinafter referred to as "SAMPLE", had the coversheet carefully removed and the present invention substrate put in its place, as the coversheet. This was to insure that the same materials were used, with the exception of the coversheets. The sanitary napkins were comprised of an absorbent material encased in a coversheet of plastic film. The strike-through values were arrived at by first subjecting "A", which had its original coversheet, to the following: the napkin was weighed and then 15.0 ml of fluid was deposited onto the coversheet of the napkin. The time it took for the fluid to pass through the coversheet into the absorbent portion of the napkin was measured. The strike-through time was then recorded. The "SAMPLE", with the present invention coversheet in place, was subjected to the same test with the strike-through time also being recorded. It should be noted that the strike-through values of both samples tested were within values acceptable to the consumer.

The rewet test, consisted of taking the wetted sanitary napkins, placing filter paper on the top of the coversheets and then placing a 5.0 pound weight on top of each of the filter papers. Prior to placing the filter paper on the napkins it was weighed. The weight compressed the napkin and forced some of the fluid to exit the napkin and be absorbed by the filter paper. The weight was left on each napkin for two (2) minutes. The weight and filter paper were then removed. The filter paper was then weighed, with the weight of each being recorded. The differences between the original weight and final weight were then determined. The following are the results of that comparison test.

| TEST RESULTS | | |
|---|---|---|
| PROPERTIES | "A" | "SAMPLE" |
| Weight (gsy) | 29.6 | 33.0 |
| Thickness (mils) | 15.5 | 12.0 |
| Strike-Through (sec) (coversheet) | 3.9 | 4.8 |
| Rewet (g) (dryness of the surface of coversheet) | 0.042 | 0.026 |

From the above results it should be noted that the "SAMPLE" coversheet proved to be approximately 50% dryer than "A", while having only 9 tenths of a second higher strike-through value. This is a substantial improvement over the dryness of prior art. The dryness of a coversheet is an important property, because it is of paramount importance with women in a sanitary napkin, to insure comfort. The present invention, as shown by the test results, exhibits a lower dryness factor than the prior art, thus a superior quality, and a substantial advantage over prior art.

The present embodiment, not only exhibits superior quality over prior art, but is also economical to make, because it is made in one process step.

It is not the intention of this specification to limit the present invention except to the following claims.

## Claims

1. A fibrous web-film coversheet material for disposable articles characterized by the fact that it comprises

at least one layer of thermoplastic film having apertures therein; and

at least one layer of a continuous fibrous web having a uniform thickness;

said apertures disposed in said film having peripheral edges;

said thermoplastic film and said continuous fibrous web disposed in a surface to surface relationship;

said apertures being disposed in said film without affecting the fibers or the uniform thickness of the fibrous web; said film and web being secured together at said peripheral edges of said apertures.

2. The coversheet of claim 1 characterized by the fact that the fibrous web is made of non-woven synthetic or natural fibers or any combinations thereof.

3. The coversheet of claim 1 characterized by the fact that the thickness of said thermoplastic film is at least 0.1 mil.

4. The coversheet of claim 1 characterized by the fact that the open area of said coversheet is at least 1%.

5. The coversheet of claim 1 characterized by the fact that the weight of the fibrous web is at least 4 grams per square yard.

6. The coversheet of claim 1 characterized by the fact that said thermoplastic film is in a heat softened state.

7. The coversheet of claim 1 characterized by the fact that said apertures cover a majority of the surface of said film.

8. The coversheet of claim 1 characterized by the fact that said apertures are positioned in zones.

9. The coversheet of claims 1, 7 or 8 characterized by the fact that the apertured area of said coversheet is 45%.

6

FIG. 1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-3 307 545 (SUROWITZ) <br> * figures 1-3; column 3, line 75 - column 4, line 8 * | 1 | A 61 F 13/00 <br> A 61 F 13/15 |
| A | | 2,3,7 | |
| Y | DE-A-2 925 090 (JOHNSON & JOHNSON) <br> * claims 1,10; figures 1,2 * | 1 | |
| A | | 2,6,7 | |
| A | US-A-2 923 298 (DOCKSTADER et al.) <br> * claims 1,3,5; figures 1-6 * | 1 | |
| A | US-A-3 441 021 (ENDRES) <br> * figures 2,3 * | 1 | |
| A | EP-A-0 275 353 (B.F. GOODRICH COMP.) <br> * claim 1; figures 1,2; column 11, lines 12-28 * | 1 | |
| D,A | US-A-3 292 619 (EGLER) <br> * figures 1-3 * | 1 | |

TECHNICAL FIELDS SEARCHED (Int. Cl.5)

A 61 F 13/00

The present search report has been drawn up for all claims

| Place of search | Date of completion of the search | Examiner |
|---|---|---|
| BERLIN | 21-04-1989 | KANAL P K |